# EUROPÄISCHE PATENTSCHRIFT

(11) **EP 0 334 167 B1**
(45) Veröffentlichungstag und Bekanntmachung des Hinweises auf die Patenterteilung: **19.05.1993**
(21) Anmeldenummer: 89104502.3
(22) Anmeldetag: 14.03.1989
(51) Int. Cl.: C08L 5/04, C08L 33/04, A61K 9/22

(54) **Feste Arzneiform, enthaltend Gallopamil**
Solid medicament containing Gallopamil
Médicament solide comprenant du Gallopamil

(30) Priorität: 23.03.1988 DE 3809764
(43) Veröffentlichungstag der Anmeldung: 27.09.1989
(73) Patentinhaber: KNOLL AG, D-67061 Ludwigshafen (DE)
(72) Erfinder: Einig, Heinz, Dr., D-6730 Neustadt 13 (DE); Stieren, Baerbel, Dr., D-6800 Mannheim 1 (DE); Buehler, Volker, Dr., D-7500 Karlsruhe (DE); Hollmann, Matthias, Dr., D-6714 Weisenheim am Sand (DE)
(74) Vertreter: Karau, Wolfgang, Dr.

(56) Entgegenhaltungen:
- EP-A- 0 063 266
- EP-A- 0 159 604
- EP-A- 0 213 083
- EP-A- 0 290 229
- DE-A- 2 820 851

## Beschreibung

Die vorliegende Erfindung betrifft eine feste Arzneiform aus einem Alginat und einem Polyacrylat sowie deren Verwendung bei der Herstellung eines Depot-Arzneimitteln.

Es ist bereits bekannt, daß man Alginate zur Herstellung von Depot-Arzneimitteln verwenden kann (Pharm. Ind. 33, 296 (1971)). Die Verwendung von Alginaten für diesen Zweck bereitet jedoch oft Schwierigkeiten. So erreichen die Formulierungen beim Tablettieren häufig keine genügende Härte. Ihr Abrieb ist daher sehr groß, was bei einem anschließenden Filmcoating stört. Mangelnde Härte führt beim Konfektionieren auf Abfüllmaschinen zu Bruch. Weiter absorbieren Alginate leicht Wasser, was zum Quellen der Tabletten oder bei Filmtabletten zum Platzen der Filme führt, wenn nicht durch eine aufwendige und teure Verpackung ein vollkommener Schutz vor Feuchtigkeit gewährleistet ist.

In der EP-A 0 063 266 ist ein Granulat beschrieben, welches Verapamil und Eudragit® RL enthält.

Gemäß der EP-A 0 159 604 lassen sich Polyacrylsäure und deren Salze zusammen mit Alginsäure oder deren Salze zur Herstellung von Arzneimitteln mit Wirkstoffen herstellen, die durch die Mundschleimhaut aufgenommen werden.

Konventionelle Retardtabletten auf Alginatbasis zeigen trotz einer in-vitro-Freisetzung nullter Ordnung keine Plasmaspiegel mit ausgeprägtem Plateaucharakter, und trotz deutlicher Verzögerung des Zeitpunkts der maximalen Plasmakonzentration gegenüber Arzneiformen, die den Wirkstoff schnell freisetzen, immer noch einen ausgeprägten und nicht immer erwünschten Plasmapeak.

Es wurde nun gefunden, daß sich diese Schwierigkeiten vermeiden lassen, wenn man den Alginaten ein bestimmtes Polyacrylat zumischt.

Gegenstand der Erfindung ist eine Arzneiform enthaltend Gallopamil und eine Mischung aus einem Alginat und Eudragit® RS im Verhältnis von 9:1 bis 2:1.

Als Alginate eignen sich besonders: Propylenglykolalginate, sowie Akali-und Erdalkalialginate, insbesondere die Natrium-, Kalium-, Ammonium- und Calciumalginate. Diese Alginate sind beispielsweise beschrieben in dem Buch von Roy L. Whistler "Industrial Gums" New York, 1973, Unterkapitel McNeely and Pettitt "über Alginate". Sie finden hauptsächlich Verwendung als Gelbildner oder Verdickungsmittel in der Lebensmitteltechnologie, Druck-, Textil- und Papierindustrie sowie bei Schweißelektroden.

Eudragit® RS ist im Datenblatt Info RS-2 der Röhm Pharma GmbH beschrieben. Es ist ein Copolymerisat aus Acryl- und Methacrylsäureestern mit einem geringen Gehalt an quartären Ammoniumgruppen:

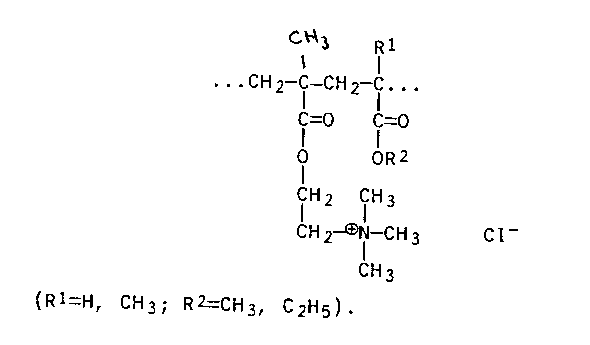

Die Verwendung der erfindungsgemäßen Mischung bietet folgende Vorteile:
1. Die Wirkstofffreisetzung aus der Arzneiform läuft weitgehend linear ab.
2. Die Tablettenkerne sind sehr hart und abriebfest, so daß sie ohne Schwierigkeiten weiterverarbeitet werden können.
3. Die Hygroskopizität der Alginate wirkt sich nicht mehr nachteilig bei der Tablettenherstellung aus. Die Tablettiermischungen können direkt ohne Granulierung verpreßt werden, und die aufwendige und komplizierte Naßgranulierung läßt sich vermeiden. Filmtabletten benötigen keinen besonderen Feuchtigkeitsschrutz mehr und platzen nicht mehr bei kurzfristiger offener Lagerung.
4. Nach Gabe der neuen Arzneiformen zeigen die Wirkstoff-Plasmaspiegel einen ausgeprägten Plateaucharakter und einen wesentlich flacheren Verlauf als nach Gabe von Arzneiformen, die nur auf Alginatbasis hergestellt sind.

Die folgenden Beispiele sollen die Erfindung näher erläutern.

### Beispiel 1

Es wurden in üblicher Weise Tabletten folgender Zusammensetzung (in mg) hergestellt:

| | A | B |
|---|---|---|
| Gallopamil·HCl | 100 | 100 |
| Na-Alginat | 240 | 332 |
| ®Eudragit RS | 92 | 0 |
| PVP | 13 | 13 |
| Mg-Stearat | 4 | 4 |
| Cellulosepulver | 31 | 31 |

Dazu wurden Gallopamil·HCl, Na-Alginat und ®Eudragit RS trocken in einem Mischer gemischt, unter Rühren mit einer wäßrigen Lösung von PVP oder PVP/PVA-Copolymerisaten (®Kollidon 30, ®kollidon 25 oder ®Kollidon VA 64) befeuchtet, durch ein Sieb getrieben und in einem Wirbelschichttrockner getrocknet, erneut über ein Sieb gegeben und mit Mg-Stearat und Cellulosepulver vermischt. Die Mischungen wurden zu Tabletten mit einem Gewicht von 480 mg verpreßt.

Die Tabletten wurden auf Härte mit dem ®Pharmatest-Härtetester und auf Abrieb mit dem ®Pharmatest-Friabilator (400 Umdrehungen in 13 min) untersucht.

Dabei wurden folgende Ergebnisse erhalten:

| Tabletten | A | B |
|---|---|---|
| Härte (N) | 122 | 61 |
| Abrieb (%) | 0,1 | 3,8 |

### Beispiel 2

Preßt man die Mischungen A und B des Beispiels 1 direkt ohne Granulierung zu Tabletten, so erhält man Preßlinge, deren Härte zwischen 70 und 110 N liegt. Die Mischung B liefert jedoch unbrauchbare Preßlinge mit einer Härte von nur 10 N.

### Beispiel 3

Es wurden analog Beispiel 1 Tabletten folgender Zusammensetzung (in mg) hergestellt:

| | C | D |
|---|---|---|
| Gallopamil | 100 | 100 |
| Na-Alginat | 267 | 267 |
| ®Eudragit RS | 67 | - |
| PVP | 13 | 13 |
| Mg-Stearat | 4 | 4 |
| Cellulosepulver | 29 | 36 |

Die Tabletten C besaßen eine Härte von 120 N, die Tabletten D von 78 N. Die entsprechenden Werte für die Friabilität betrugen 0,05 % bzw. 2,1 %.

Preßt man die Tabletten direkt, d.h. ohne Granulierung, so erhält man Tabletten mit der Härte 100 N (C) bzw. 13 N (D).

### Testung der Tabletten

Testpersonen erhielten entweder je 1 Tablette C oder D (vgl. Beispiel 3). Anschließend wurden in bestimmten Zeitabständen Blutproben entnommen und der Gehalt an Gallopamil darin ermittelt:

| | | | | | | | | | |
|---|---|---|---|---|---|---|---|---|---|
| Zeit (h) | 0,5 | 1 | 2 | 3 | 4 | 6 | 8 | 10 | 12 |
| C (ng/ml Plasma) | 0,7 | 0,9 | 4,6 | 6,8 | 10 | 9,0 | 8,9 | 8,2 | 5,9 |
| D (ng/ml Plasma) | 3,8 | 10 | 29 | 33 | 36 | 17 | 10 | 6,4 | 6,9 |

Im Gegensatz zu D zeigt C einen wesentlich flacheren Verlauf der Plasmawerte.

## Patentansprüche (Patentansprüche für folgende(n) Vertragsstaat(en): AT, BE, CH, DE, FR, GB, IT, LI, NL, SE)

1. Feste Arzneiform enthaltend Gallopamil und eine Mischung aus einem Alginat und ®Eudragit RS, einem Copolymerisat aus Acryl- und Methacrylsäureestern mit einem geringen Gehalt an quartären Ammoniumgruppen, im Verhältnis 9:1 bis 2:1.

## Patentansprüche (Patentansprüche für folgende(n) Vertragsstaat(en): ES)

1. Verfahren zur Herstellung einer festen Arzneiform, enthaltend Gallopamil und eine Mischung aus einem Alginat und ®Eudragit RS, einem Copolymerisat aus Acryl- und Methacrylsäureestern mit einem geringen Gehalt an quartären Ammoniumgruppen, dadurch gekennzeichnet, daß man das Alginat und das Eudragit RS im Verhältnis von 9:1 bis 2:1 miteinander mischt und mit dem Gallopamil zu einer festen Arzneiform verarbeitet.

## Claims (Claims for the following Contracting State(s): AT, BE, CH, DE, FR, GB, IT, LI, NL, SE)

1. A solid drug containing gallopamil and a mixture of an alginate and ®Eudragit RS, a copolymer of acrylates and methacrylates having a low content of quaternary ammonium groups, in a ratio of from 9 : 1 to 2 : 1.

## Claims (Claims for the following Contracting State(s): ES)

1. A process for the preparation of a solid drug containing gallopamil and a mixture of an alginate and ®Eudragit RS, a copolymer of acrylates and methacrylates having a low content of quaternary ammonium groups, wherein the alginate and the Eudragit RS are mixed with one another in a ratio of from 9 : 1 to 2 : 1 and processed with the gallopamil to give a solid drug.

## Revendications (Revendications pour l'(les) Etat(s) contractant(s) suivant(s): AT, BE, CH, DE, FR, GB, IT, LI, NL, SE)

1. Médicament solide contenant du gallopamile et un mélange d'un alginate et d'Eudragit® RS, un copolymérisat d'acrylate et méthacrylate à faible teneur en groupes d'ammonium quaternaire, en rapport de 9/1 à 2/1.

## Revendications (Revendications pour l'(les) Etat(s) contractant(s) suivant(s): ES)

1. Procédé de préparation de médicament solide contenant du gallopamile et un mélange d'un alginate et d'Eudragit® RS, un copolymérisat d'acrylate et méthacrylate à faible teneur en groupes d'ammonium quaternaire, caractérisé par le fait qu'on mélange l'alginate et l'Eudragit RS dans un rapport de 9/1 à 2/1 et on le transforme en médicament solide avec le gallopamile.
